Europäisches Patentamt

**(19)** European Patent Office

Office européen des brevets

**(11)** **EP 0 526 526 B1**

**(12)** **EUROPEAN PATENT SPECIFICATION**

**(45)** Date of publication and mention
of the grant of the patent:
**26.06.1996 Bulletin 1996/26**

**(21)** Application number: **91908447.5**

**(22)** Date of filing: **08.04.1991**

**(51)** Int. Cl.$^6$: **A23D 7/00**

**(86)** International application number:
**PCT/US91/02272**

**(87)** International publication number:
**WO 91/15960 (31.10.1991 Gazette 1991/25)**

**(54) NOVEL SOLID, NONDIGESTIBLE, FAT-LIKE COMPOUNDS AND FOOD COMPOSITIONS CONTAINING SAME**

NEUE FESTE UNVERDAULICHE FETTÄHNLICHE VERBINDUNGEN UND DIESE ENTHALTENDE NAHRUNGSMITTELZUSAMMENSETZUNGEN

NOUVEAUX COMPOSES SOLIDES, NON DIGESTIBLES, ANALOGUES A DE LA MATIERE GRASSE ET COMPOSITIONS ALIMENTAIRES LES CONTENANT

**(84)** Designated Contracting States:
**AT BE CH DE DK ES FR GB GR IT LI LU NL SE**

**(30)** Priority: **26.04.1990 US 514794**

**(43)** Date of publication of application:
**10.02.1993 Bulletin 1993/06**

**(73)** Proprietor: **THE PROCTER & GAMBLE COMPANY**
**Cincinnati, Ohio 45202 (US)**

**(72)** Inventors:
• **LETTON, James, Carey**
  **Forest Park, OH 45240 (US)**
• **BACK, Deborah, Jean**
  **Cleves, OH 45002 (US)**
• **BAGINSKI, John, Robert**
  **Loveland, OH 45140 (US)**

• **ELSEN, Joseph, James**
  **Cincinnati, OH 45211 (US)**
• **GUFFEY, Tymothy, Bruce**
  **West Chester, OH 45069 (US)**
• **KESTER, Jeffrey, John**
  **West Chester, OH 45069 (US)**
• **WEISGERBER, David, John**
  **Cincinnati, OH 45239 (US)**

**(74)** Representative: **Canonici, Jean-Jacques**
**Procter & Gamble European Technical Center N.V.**
**Temselaan 100**
**1853 Strombeek-Bever (BE)**

**(56)** References cited:
**EP-A- 0 290 421**          **US-A- 3 600 186**

Printed by Rank Xerox (UK) Business Services
2.12.4/3.4

**Description**

FIELD OF THE INVENTION

The invention pertains to novel, nondigestible, solid fat-like compounds which are particularly useful as additives to liquid edible oils. They can be used in blends with liquid nondigestible oils to control passive oil loss through the anal sphincter when said oils are ingested. They can also be used as non-caloric thickening agents with liquid digestible or nondigestible oils to formulate fluid cooking and salad oils or semi-solid oleaginous products such as shortening and margarines.

BACKGROUND ART

In recent years considerable attention has been focused on the amount of triglyceride fat in the diet from the standpoint of health concerns about obesity and hyper-cholesterolemia. Numerous patents have been directed to providing materials which have the physical and gustatory characteristics of triglyceride fats, but which are absorbed to a low extent or not at all by the body. These materials are referred to variously as noncaloric fats, pseudofats, nondigestible fats and fat substitutes. Patents pertaining to such materials include U.S. Pat. Nos. 4,582,927, Fulcher, issued April 15, 1986, (fatty esters of malonic acid); 4,582,715, Volpenhein, issued April 15, 1986, (alpha acetylated triglycerides); and 3,579,548, Whyte, issued May 18, 1981, (triglycerides of alpha-branched chain carboxylic acids).

One particular type of compound which has achieved considerable attention as a nondigestible fat is sucrose polyester (i.e., sucrose in which at least four of the eight hydroxyl groups are esterified with a fatty acid). U.S. Pats. 3,600,186, Mattson, issued Aug. 17, 1971; 4,368,213, Hollenbach et al. issued January 11, 1983; and 4,461,782, Robbins et al. issued July 24, 1984 describe the use of this material as a nondigestible fat in a variety of food compositions.

A problem associated with moderate to high levels of ingestion of liquid nondigestible fats, i.e., those having a melting point below body temperature (about 37°C), is an undesired passive oil loss effect, which is manifested in leakage of the liquid nondigested fat through the anal sphincter. U.S. Pat. No. 4,005,195, Jandacek, issued Jan. 25, 1977, discloses the combining of higher melting fatty materials such as solid triglycerides and solid sucrose polyesters with the liquid sucrose polyesters in order to avoid the oil loss effect. EP 210 421 describes a mixture of intermediate melting polyol polyesters with a triglyceride hardstock.

U.S. Pat. 4,797,300 (Jandacek et al.), issued January 10, 1989 discloses the use of certain solid sucrose polyesters which have high oil binding capacity for liquid sucrose polyesters and liquid triglycerides, when used at levels of 10% to 25% in said oils. It is disclosed that because of their high oil binding capacity, these solid sucrose polyesters have outstanding utility as agents to control or prevent passive oil loss of liquid nondigestible sucrose polyesters, and they are also useful as non-caloric hardstocks to use with liquid digestible or nondigestible oils in the preparation of semi-solid fat products such as shortenings and margarines. The oil binding agents of the Jandacek et al. '300 patent are solid sucrose polyesters wherein the ester groups consist essentially of a mixture of short chain saturated fatty acid ester radicals ($C_2$-$C_{10}$) and long chain saturated fatty acid radicals ($C_{20}$-$C_{24}$) in a molar ratio of short chain to long chain of from 3:5 to 5:3, and wherein the degree of esterification is from 7 to 8.

U.S. Pat. 3,158,490 (Baur et al.), issued November 24, 1964 discloses sucrose (and other disaccharide) polyesters which are useful as additives at 0.001% to 0.5% level in triglyceride salad oils to prevent clouding in low-temperature storage of the oils. The degree of esterification is at least 3, i.e., no more than 5 of the 8 hydroxyl groups are unesterified. The ester groups are a combination of: (1) from 15-85% saturated $C_{14}$-$C_{22}$ fatty acids, and (2) the balance selected from saturated $C_2$-$C_{12}$ or unsaturated $C_{14}$-$C_{22}$ fatty acids. Arachidic ($C_{20}$) and behenic ($C_{22}$) acids are recited as specific examples of (1) and acetic ($C_2$), caprylic ($C_8$), and oleic ($C_{18-1}$) acids are recited as specific examples of (2). At col. 2, lines 5-10, a sucrose ester having 2 oleic and 6 palmitic groups is disclosed, and it is stated that long chain saturated acids such as myristic, stearic, arachidic, behenic, or mixtures thereof can be used in place of all or part of the palmitic.

It is an object of the present invention to provide novel solid nondigestible fat materials which are suitable substitutes for solid fat in foods.

Another object of the present invention is to provide novel, solid, nondigestible fat materials which are especially effective oil binding agents for use in mixture with liquid nondigestible oils in food products to control or prevent passive oil loss of the liquid oils when ingested.

It is another objective of the invention to provide novel solid nondigestible fat materials which are effective in binding oils and are thereby especially useful in formulating shortenings and other semi-solid products and fluid cooking and salad oils from liquid digestible or nondigestible oils.

For purposes of describing this invention, the term "nondigestible" shall mean being absorbable to an extent of only 70% or less (especially 20% or less) by the human body through its digestive system.

All percentages and proportions herein are "by weight" unless otherwise specified.

# EP 0 526 526 B1

## SUMMARY OF THE INVENTION

The invention is directed to novel solid polyol polyesters wherein the polyol has at least 4 hydroxyl groups, the ester groups comprise a combination of: (i) long chain (at least 12 carbon atoms) unsaturated fatty acid radicals, or a mixture of said radicals and saturated short chain ($C_2$-$C_{12}$) fatty acid radicals, and (ii) long chain (at least 20 carbon atoms) saturated fatty acid radicals, in a molar ratio of (i):(ii) of from about 1:15 to about 2:1, and wherein at least 4 of the hydroxyl groups of the polyol are esterified.

## DETAILED DESCRIPTION OF THE INVENTION

It has now been found that certain polyol polyesters which are solid at temperatures of 25°C and higher, in addition to being suitable nondigestible substitutes for solid fat in the diet, are highly effective thickening agents for triglyceride oils and nondigestible oils such as liquid polyol polyesters. Accordingly, these solid polyol fatty acid polyesters can be used as "thickening agents" or "hardstocks" for blending with liquid digestible or nondigestible oils in the formulation of cooking and salad oils or semi-solid fat products such as shortenings, as well as other food products which contain a combination of fat and non-fat ingredients, e.g., margarines, mayonnaise, frozen dairy desserts and the like. Further, this high capacity to thicken liquid oils makes compounds of the invention having a melting point above body temperature (37°C) particularly useful in the formulation of food products containing the nondigestible oils so as to control or prevent the passive oil loss problem associated with the ingestion of such oils.

The novel solid polyol fatty acid polyesters of the present invention are polyol polyesters wherein the ester groups comprise a combination of: (i) long chain, unsaturated fatty acid radicals or a mixture of long chain unsaturated fatty acid radicals and short chain saturated fatty acid radicals, and (ii) long chain saturated fatty acid radicals, the ratio of (i):(ii) being from 1:15 to 2:1, and wherein at least 15% (preferably at least about 30%, more preferably at least 50%, and most preferably at least 60%) by weight of the total fatty acid radicals in the solid polyol polyester are $C_{20}$ or higher saturated fatty acid radicals. The long chain unsaturated fatty acid radicals are typically straight chain (i.e., normal) and contain at least 12 (preferably about 12 to 26, more preferably 18 to 22) carbon atoms. The most preferred unsaturated radicals are the $C_{18}$ mono and/or diunsaturated fatty acid radicals. The short chain saturated fatty acid radicals are typically normal and contain 2 to 12 (preferably 6 to 12 and most preferably 8 to 12) carbon atoms. The long chain saturated fatty acid radicals are typically normal and contain at least 20 (preferably 20 to 26, most preferably 22) carbon atoms. The molar ratio of Group (i) fatty acid radicals to Group (ii) fatty acid radicals in the polyester molecule is from 1:15 to 2:1 (preferably 1:7 to 5:3, more preferably 1:7 to 3:5). A typical suitable range is 3:5 to 4:4. The average degree of esterification of these solid polyol fatty acid polyesters is such that at least 4 of the hydroxyl groups of the polyol are esterified. In the case of sucrose polyesters from about 7 to 8 of the hydroxyl groups of the polyol are preferably esterified. Typically, substantially all (e.g., at least 85%, preferably at least 95%) of the hydroxyl groups of the polyol are esterified.

The polyols which are used in the solid polyol polyester compounds of the present invention preferably contain from 4 to 11 (more preferably 4 to 8, most preferably 6 to 8) hydroxyl groups.

Examples of preferred polyols are sugars (including monosaccharides and disaccharides and trisaccharides) and sugar alcohols, containing from 4 to 11 hydroxyl groups. The trisaccharides raffinose and maltotriose are examples of sugars which contain 11 hydroxyl groups. The preferred sugars and sugar alcohols are those which contain 4 to 8 (more preferably 6 to 8) hydroxyl groups. Examples of those containing four hydroxyl groups are the monosaccharides xylose and arabinose and the sugar alcohol erythritol. Suitable five hydroxyl group-containing polyols are the monosaccharides galactose, fructose, mannose and glucose, and the sugar alcohol xylitol. A polyol containing six hydroxyl groups is sorbitol. Examples of disaccharide polyols which can be used include maltose, lactose, and sucrose, all of which contain eight hydroxyl groups.

Examples of other suitable polyols are pentaerythritol, diglycerol, triglycerol, alkyl glycosides, and polyvinyl alcohols. The preferred polyol is sucrose.

Examples of long chain unsaturated fatty acid radicals for the solid polyol polyesters herein are lauroleate, myristoleate, palmitoleate, oleate, elaidate, erucate, linoleate, linolenate, arachidonate, eicosapentaenoate, and docosahexaenoate. For oxidative stability, the mono- and diunsaturated fatty acid radicals are preferred.

Examples of suitable short chain saturated fatty acid radicals are acetate, caproate, caprylate, caprate and laurate. Examples of suitable long chain saturated fatty acid radicals are arachidate, behenate, lignocerate, and cerotate.

Of course, the long chain unsaturated fatty acid radicals can be used singly or in mixtures with each other or in mixtures with the short chain saturated fatty acid radicals, in all proportions. Likewise, the long chain saturated acid radicals can be used in combination with each other in all proportions. Mixed fatty acid radicals from source oils which contain substantial amounts of the desired unsaturated or saturated acids can be used as the fatty acid radicals to prepare compounds of the invention. The mixed fatty acids from the oils should contain at least 30% (preferably at least 50%, and most preferably at least 80%) of the desired unsaturated or saturated acids. For example, rapeseed oil fatty acids or soybean oil fatty acids can be used instead of pure $C_{12}$-$C_{26}$ unsaturated fatty acids. Hardened (i.e., hydrogenated) high erucic rapeseed oil fatty acids can be used instead of pure $C_{20-26}$ saturated acids. Preferably the $C_{20}$ and

higher acids (or their derivatives - e.g., methyl esters) are concentrated, for example by distillation. The fatty acids from palm kernel oil or coconut oil can be used as a source of $C_8$ to $C_{12}$ acids. An example of the use of source oils to make solid polyol polyesters of the invention is the preparation of solid sucrose polyester, employing the fatty acids of high oleic sunflower oil and substantially completely hydrogenated high erucic rapeseed oil. When sucrose is substantially completely esterified with a 1:3 by weight blend of the methyl esters of the fatty acids of these two oils, the resulting sucrose polyester will have a molar ratio of unsaturated $C_{18}$ acid radicals to $C_{20}$ and higher saturated acid radicals of 1:1 and 28.6 weight percent of the total fatty acids in the polyester will be $C_{20}$ and $C_{22}$ fatty acids.

The higher the proportions of the desired unsaturated and saturated acids in the fatty acid stocks used in making the solid polyol polyester, the more efficient the ester will be in its ability to bind liquid oils.

The preferred unsaturated fatty acid radicals are those which have 18 carbon atoms, and are mono- and/or diunsaturated. Preferred short chain fatty acid radicals are those which have 8-12 carbon atoms. The preferred long chain saturated fatty acid radical is behenate. The preferred ratio of Group (i) fatty acid radicals to Group (ii) fatty acid radicals is from 1:7 to 5:3 (more preferably 1:7 to 3:5). Preferred solid polyol polyesters of the invention are polyesters of sucrose in which at least 7 of the 8 hydroxyl groups are esterified.

Examples of solid polyol polyesters of the present invention are sorbitol hexaester in which the acid ester radicals are palmitoleate and arachidate in a 1:2 molar ratio; the octaester of raffinose in which the acid ester radicals are linoleate and behenate in a 1:3 molar ratio; the heptaester of maltose wherein the esterifying acid radicals are sunflower seed oil fatty acids and lignocerate in a 3:4 molar ratio; the octaester of sucrose wherein the esterifying acid radicals are oleate and behenate in a 2:6 molar ratio; and the octaester of sucrose wherein the esterifying acid radicals are laurate, linoleate and behenate in a 1:3:4 molar ratio. A preferred material is sucrose polyester in which the degree of esterification is 7-8, and in which the fatty acid radicals are $C_{18}$ mono- and/or diunsaturated and behenic, in a molar ratio of unsaturates:behenic of 1:7 to 3:5.

The fatty acid composition (FAC) of the polyol polyesters is determined by gas chromatography, using a Hewlett-Packard Model S712A gas chromatograph equipped with a thermal conductivity detector and a Hewlett-Packard Mode 17671A automatic sampler. The chromatographic method used is described in <u>Official Methods and Recommended Practices of the American Oil Chemists Society</u>, 3rd Ed., 1984, Procedures 1-$C_e$62 .

The solid polyol polyesters of the present invention can be made according to prior known methods for preparing polyesters of polyols. Since the sucrose polyesters are the preferred solid polyol polyesters herein; the invention will be exemplified primarily by these materials. One such method of preparation is by reacting the acid chlorides of the fatty acids with sucrose. In this method a mixture of the Group (i) and Group (ii) acid chlorides can be reacted in one step with sucrose, or the Group (i) and Group (ii) acid chlorides can be reacted sequentially with sucrose. Acid anhydrides can be used instead of acid chlorides. Another preparation method is by the process of reacting methyl esters of the fatty acids with sucrose in the presence of a fatty acid soap and a basic catalyst such as potassium carbonate. See, U.S. Pat. Nos. 4,797,300, Jandacek et al, issued January 10, 1989, 3,963,699, Rizzi et al., issued June 15, 1976; 4,518,772, Volpenhein, issued May 21, 1985; and 4,517,360, Volpenhein, issued May 14, 1985, and USSN 417,990, Letton, filed October 6, 1989,

When using the methyl ester route for preparing the solid polyol polyesters herein in which the Group (i) fatty acids are the long chain unsaturated acids, the saturated long chain and unsaturated long chain methyl esters are blended in the desired ratio and reacted with sucrose by transesterification to obtain the sucrose esters of mixed unsaturated/saturated fatty acids. In a preferred way of practicing the methyl ester process, five moles of the blended long chain saturated/long chain unsaturated methyl esters are reacted with sucrose in a first stage at 135°C to obtain partial esters of sucrose. An additional nine moles of the blended esters are then added and the reaction continued at 135°C under reduced pressure until the desired degree of esterification has been attained.

The solid polyol polyesters of the present invention have complete melting points above 25°C, preferably above 37°C, more preferably above 50°C and most preferably above about 60°C. Melting points reported herein are measured by Differential Scanning Calorimetry (DSC). These solid materials have the ability to trap relatively large amounts of oil within their crystal structure. As a consequence, they can be used as "hardstocks" by blending them in amounts of about 1% to 50% (typically 1% to 25%) with liquid oils to prepare semi-solid compositions such as shortenings and margarines. A typical suitable range is from 10% to 25%. The oils for these compositions can be conventional digestible triglyceride oils such as cottonseed, corn, canola, or soybean oil, or nondigestible edible oils. The solid polyol polyesters of the invention having complete melting points above 37°C can be blended at levels of as low as about 1% (preferably at least 2%) with liquid nondigestible oils having complete melting points below 37°C in order to control passive oil loss upon ingestion of food compositions containing the nondigestible oil.

Examples of nondigestible edible oils which can be used in compositions of the invention are liquid polyesters of sugars and sugar alcohols (U.S. Pat. No. 4,005,195, Jandacek, issued January 25, 1977); liquid esters of tricarballylic acids (U.S. Pat. No. 4,508,746, Hamm, issued April 2, 1985); liquid diesters of dicarboxylic acids such as derivates of malonic and succinic acid (U.S. Pat. No. 4,582,927, Fulcher, issued April 15, 1986); liquid triglycerides of alpha-branched chain carboxylic acids (U.S. Pat. No. 3,579,548, Whyte, issued May 18, 1971); liquid ethers and ether esters containing the neopentyl moiety (U.S. Pat. No. 2,962,419, Minich, issued Nov. 29, 1960; liquid fatty polyethers of polyglycerol (U.S.

Pat. No. 3,932,532, Hunter et al., issued Jan. 13, 1976); liquid alkyl glycoside polyesters (U.S. Pat. 4,840,815 to Meyer et al., issued June 20, 1989); liquid polyesters of two ether-linked hydroxycarboxylic acids (e.g., citric or isocitric) (U.S. Pat. 4,888,195 to Huhn et al., issued December 19, 1988); liquid esters of epoxide-extended polyols (U.S. Pat. 4,861,613 to White et al, issued August 29, 1989);. Edible polydimethyl siloxanes (e.g., Fluid Silicones available from Dow-Corning Corporation) constitute another type of nondigestible oil which can be used in the compositions herein.

The preferred nondigestible oils are the liquid (complete melting point below 37°C, preferably below 25°C) fatty acid polyesters of sugars or sugar alcohols having at least 4 hydroxyl groups, wherein at least 4 of the hydroxyl groups are esterified. The preferred polyol is sucrose. Examples are the hexa, hepta, and octaesters of sucrose. Examples of esterifying acid groups for these esters are soybean, cottonseed, sunflower seed, coconut, and palm kernel acids (see U.S. Pat. 4,005,195, supra).

The solid polyol polyesters of the present invention can be used in mixtures with other solid polyol polyesters, solid fatty acids or solid triglycerides such as disclosed in U.S. Pat. 4,005,195 (Jandacek), issued January 25, 1977, in order to control oil loss resulting from ingestion of nondigestible liquid polyol polyesters. They can also be combined with intermediate melting mixtures of liquid and solid nondigestible polyol polyesters such as those disclosed in U.S. Pat. 4,880,657 (Guffey et al.), issued November 14, 1989.

When substituting nondigestible oils for fat in foods which contain fat and non-fat ingredients (e.g., starches, sugar, non-fat milk solids, etc.) the solid polyol polyesters are included to control passive oil loss when said foods are ingested. In such products the mixture of solid polyol polyester of the invention and nondigestible oil is substituted for up to 100% of the fat normally present in such foods. The weight ratio of liquid nondigestible oil to solid polyol polyester will typically be in the range of from 99:1 to 1:1, or alternatively from 99:1 to 3:1. A typical suitable range is from 8.9:1 to 3:1.

The mixtures of solid polyol polyesters of the invention and liquid digestible or nondigestible oils are typically prepared by simply mixing the two materials together, typically at a temperature above the melting point of the solid polyol polyesters.

When using a particularly preferred sucrose polyester of the invention, wherein the unsaturated fatty acid radical is $C_{18}$ mono- and/or diunsaturated and the saturated fatty acid radical is behenic in a molar ratio of 1:7 to 3:5, the preferred ratio of liquid nondigestible oil to solid sucrose polyester is from 99:1 to 9:1, preferably 99:1 to 94:6.

Mixtures of solid polyol polyesters of the invention with edible nondigestible oils are further characterized in having a relatively flat solids content profile across the temperature range of from typical room temperature to body temperature, i.e., from 21.1°C (70°F) to 37°C (98.6°F). The slope of the SFC curve is expressed as the change in percent solids per unit change in temperature, in °F. Typically the slope of the Solid Fat Content (SFC) between these temperatures is between 0 and -0.75. Generally, the greater the weight percent of $C_{20}$ or higher saturated fatty acid radicals in the solid polyol polyester, the flatter the SFC slope will be. For example, at the 30% $C_{20}$ or higher fatty acid level the slope will typically be between 0 and -0.5 and at 50% it will typically be between 0 and -0.3.

Determination of SFC values over a range of temperatures can be done by a method involving PNMR (Pulsed Nuclear Magnetic Resonance). Such method is well known to those skilled in the art (see J. Amer. Oil Chem. Soc., Vol. 55 (1978), pp. 328-31, and A.O.C.S. Official Method Cd. 16-81, Official Methods and Recommended Practices of The American Oil Chemists Society, 3rd. Ed., 1987;.

Before determining the SFC values, a sample of the fat composition is heated to a temperature of 140°F (60°C) or higher for at least 30 minutes or until the sample is completely melted. The melted sample is then tempered as follows: at 80°F (26.7°C) for 15 minutes; at 32°F (0°C) for 15 minutes; at 80°F (26.7°C) for 30 minutes; at 32°F (0°C) for 15 minutes. After tempering, the SFC values of the sample at temperatures of 50°F (10°C), 70°F (21.1°C), 80°F (26.7°C), 92°F (33.3°C), and 98.6°F (37°C) are determined by PNMR after equilibration for 30 minutes at each temperature. The slope of the SFC profile is calculated by substracting the percent solids at 70°F from the percent solids at 98.6°F and dividing that value by 28.6.

The nondigestible solid fat materials herein can be used in combination with other nondigestible or digestible fats and oils to make shortening and oil products. The other fats and oils can be synthetic or derived from animal or vegetable sources, or combinations of these. These shortenings and oils can be used in frying applications such as preparation of french fry potatoes, potato chips, corn chips, tortilla chips, donuts, chicken, fish, and fried pies (e.g., turnovers).

These shortenings and oils can also be used in the production of baked goods in any form, such as mixes, shelf-stable baked goods, and frozen baked goods. Applications include, but are not limited to, cakes, granola bars, brownies, muffins, bar cookies, wafers, biscuits, pastries, pies, pie crusts, and cookies, including sandwich cookies and chocolate chip cookies, particularly the storage-stable dual-textured cookies described in U.S. Pat. No. 4,455,333 of Hong & Brabbs. The baked goods can contain fruit, cream, or other fillings. Other baked good uses include breads and rolls, crackers, pretzels, pancakes, waffles, ice cream cones and cups, yeast-raised baked goods, pizzas and pizza crusts, and baked farinaceous snack foods, and other baked salted snacks.

The solid polyol polyesters herein can also be used as a component of the fat portion of many other foods such as ice cream, frozen desserts, cheese, cheese spreads, meats, meat analogs, chocolate confections, salad dressings, mayonnaise, margarine, spreads, sour cream, yogurt, coffee creamer, peanut butter, extruded snacks, roasted nuts and beverages such as milkshakes.

The present nondigestible solid fat materials can also be fortified with vitamins and minerals, particularly the fat-soluble vitamins. The fat-soluble vitamins include vitamin A, vitamin D, and vitamin E. (See U.S. Pat. 4,034,083 (Mattson) issued July 5, 1977, .)

The present nondigestible solid fat materials (or blends thereof with edible oils) are particularly useful in combination with particular classes of food and beverage ingredients. For example, an extra calorie reduction benefit is achieved when the fat materials are used with noncaloric or reduced calorie sweeteners alone or in combination with bulking agents. Noncaloric or reduced calorie sweeteners include, but are not limited to, aspartame, saccharin, alitame, thaumatin, dihydrochalcones, acesulfame, and cyclamates.

The present nondigestible fat materials can also be used in combination with reduced calorie medium chain and mixed medium/long chain triglycerides such as are disclosed in U.S. Pat. 4,888,196, Ehrman et al. issued December 19, 1989 and European Patent Application 322,027, Seiden, published June 28, 1989,

Bulking or bodying agents are useful in combination with the nondigestible solid fat materials herein in many food compositions. The bulking agents can be nondigestible carbohydrates, for example, polydextrose and cellulose or cellulose derivatives, such as carboxymethylcellulose, carboxyethylcellulose, hydroxypropylcellulose, methylcellulose, hydroxypropyl methylcellulose, and microcrystalline cellulose. Other suitable bulking agents include gums (hydrocolloids), starches, dextrins, fermented whey, tofu, maltodextrins, polyols, including sugar alcohols, e.g., sorbitol and mannitol, and carbohydrates, e.g., lactose.

Similarly, food and beverage compositions can be made that combine the present nondigestible solid fat materials with dietary fibers to achieve the combined benefits of each. By "dietary fiber" is meant complex carbohydrates resistant to digestion by mammalian enzymes, such as the carbohydrates found in plant cell walls and seaweed, and those produced by microbial fermentation. Examples of these complex carbohydrates are brans, celluloses, hemicelluloses, pectins, gums and mucilages, seaweed extract, and biosynthetic gums. Sources of the cellulosic fiber include vegetables, fruits, seeds, cereals, and manmade fibers (for example, by bacterial synthesis). Commercial fibers such as purified plant cellulose, or cellulose flour, can also be used. Naturally occurring fibers can be used, such as psyllium and fibers from whole citrus peel, citrus albedo, sugar beets, citrus pulp and vesicle solids, apples, apricots, and watermelon rinds.

These dietary fibers may be in a crude or purified form. The dietary fiber used may be of a single type (e.g., cellulose), a composite dietary fiber (e.g., citrus albedo fiber containing cellulose and pectin), or some combination of fibers (e.g., cellulose and a gum). The fibers can be processed by methods known to the art.

Of course, judgment must be exercised to make use of the nondigestible solid fat materials and combinations thereof with other food ingredients. For example, a combination of sweetener and nondigestible solid fat material would not be used where the specific benefits of the two are not desired. The nondigestible solid fat materials and nondigestible solid fat material/ingredient combinations are used where appropriate, and in appropriate amounts.

Many benefits are obtained from the use of the present nondigestible solid fat materials in food and beverage compositions, either when used alone or in combination with edible oils and/or other ingredients discussed above. A primary benefit is the calorie reduction achieved when nondigestible fat materials are used as a total or partial fat replacement. This calorie reduction can be increased by using combinations of the present nondigestible solid fat materials with reduced calorie sweeteners, bulking agents, or other nondigestible fats and oils. Another benefit which follows from this use is a decrease in the total amount of fat and saturated fat in the diet. Foods or beverages made with the nondigestible solid fat materials instead of animal-derived triglyceride fats will also contain less cholesterol, and the ingestion of these foods can lead to reduced serum cholesterol and thus reduced risk of heart disease.

A related benefit is that the use of the nondigestible solid fat materials allows the production of foods that are stable in terms of shelf stability and penetration stability. Compositions made with these fat materials have acceptable organoleptic properties, particularly taste and texture.

Dietary foods can be made with the nondigestible solid fat materials, to meet special dietary needs, for example, of persons who are obese, diabetic, or hypercholesterolemic. The nondigestible solid fat materials can be a major part of a low-fat, low-calorie, low-cholesterol diet, and they can be used alone or in combination with drug therapy or other therapy. Combinations of food or beverage products made with the nondigestible solid fat materials can be used as part of a total dietary management regimen, based on one or more of these products, containing the fat materials alone or in combination with one or more of the above-mentioned ingredients, to provide one or more of the above-mentioned benefits.

In formulating food products comprising fat and nonfat ingredients (e.g., margarines, mayonnaise, baked goods, etc.) in which the fat component comprises a nondigestible oil (e.g., a liquid sucrose polyester such as sucrose octaoleate), the solid polyol polyesters of the present invention can be included in said products to control passive oil loss of the nondigestible oil which would otherwise occur as a result of ingestion of the products. The solid polyol polyester will generally be used in the food products at a level such that the ratio of the non-digestible oil to solid polyol polyester is from 99:1 to 1:1, more typically 99:1 to 3:1. A typical suitable range is from 9:1 to 3:1.

This discussion of the nondigestible solid fat material uses, combinations, and benefits is not intended to be limiting or all-inclusive. It is contemplated that other similar uses and benefits can be found that will fall within the spirit and scope of this invention.

In addition to food compositions, the solid polyol polyesters of the present invention can be used in formulating lubricants, skin creams, cosmetics, pharmaceuticals, and the like.

The invention will be illustrated by the following examples.

EXAMPLE I

Preparation of Tetraoleyl Tetrabehenyl Sucrose (Acid Chloride Route)

| Chemicals: | | | | |
|---|---|---|---|---|
| A. Reaction | Mol. Wt. | Wt. (g) | Moles | Mole Ratio |
| 1. Sucrose | 342.3 | 10 | 0.0292 | 1 |
| 2. Oleyl chloride | 300.47 | 38 | 0.1264 | 4.3 |
| 3. Behenyl chloride | 358 | 41.8 | 0.1168 | 4 |
| B. Solvents | | | | |
| 1. Pyridine | | | | |
| 2. Dimethylformamide | | | | |
| 3. Dichloromethane | | | | |

Procedure

10 g of sucrose are dissolved in a solution of 150 ml pyridine and 75 ml dimethylformamide by heating to about 55°C while under a nitrogen atmosphere. The solution is cooled to about 40°C and a solution of 41.8 g of behenyl chloride in 150 ml of dichloromethane is added dropwise over a period of 1 hour and 45 minutes. Temperature during the addition is maintained at about 40-44°C, and the system is also maintained under a nitrogen atmosphere.

Following addition of the behenyl chloride, the reaction is stirred at 40°C for an additional 3 hours, then cooled to 30°C. 38 g of oleyl chloride in 100 ml of dichloromethane are then added dropwise over a 45 minutes period. The reaction temperature is maintained at about 30°C during this addition period, then raised to 40°C and held at that temperature for about 1 hour and 30 minutes. Heat is then discontinued and the reaction mixture stirred at ambient temperature overnight.

The reaction mixture is then warmed to 40°C and stirred at 40°C for one hour before cooling to room temperature (about 27°C). The mixture is then filtered to remove crystalline pyridine hydrochloride and the filtrate is stripped under vacuum to remove dichloromethane, pyridine, and dimethylformamide. The distillation residue is then re-dissolved in dichloromethane and the solution transferred to a 2-liter separatory funnel.

The dichloromethane solution is then washed two times with a dilute solution of sodium chloride, then with a dilute solution of hydrochloric acid to remove residual pyridine. The dichloromethane solution is then washed two times with water, then with dilute calcium hydroxide solution. The dichloromethane/water mixture is then filtered through Celite to remove a small amount of precipitate (probably calcium salts of the acids), then the mixture is separated in a 2-liter separatory funnel. The dichloromethane solution is then washed neutral with water and the dichloromethane solution is dried over magnesium sulfate for several days. The dried mixture is then filtered and stripped under vacuum to give a residue which solidifies at room temperature. The solid polyol polyester has a hydroxyl value of 5.7 (corresponding to a calculated degree of esterification of 7.73 - about 93% of hydroxyl groups esterified). The percent octaester in the material is 83.

EXAMPLE II

Preparation of a Solid Sucrose Polyester from Methyl Esters Containing High Proportions of $C_{18}$ Unsaturates and $C_{22}$ Saturates

This example describes the preparation of solid sucrose polyesters of this invention by a modification of the process described in U.S. Pat. Nos. 4,518,772, supra, and 4,517,360, supra.

High erucic acid rapeseed oil (HEAR) is blended with low erucic acid rapeseed oil (LEAR) to a composition of 38% erucic acid. The rapeseed oil blend is mixed with 3%-6% refined, bleached cottonseed oil to obtain an oil composition having approximately 35% of $C_{22}$ acids (i.e., behenic plus erucic). This rapeseed/cottonseed stock is then hydrogenated

EP 0 526 526 B1

to an iodine value less than 4. Hydrogenation is done with nickel catalyst levels typical of those used for any vegetable oil using 0-100 psig pressure, and a temperature of approximately 375°F.

The material is deodorized at a temperature of 375-495°F. The hardened, deodorized rapeseed/cottonseed oil has the following characteristics: fatty acid composition: 3-7% $C_{16:0}$, 45-55% $C_{18:0}$, 0-2% $C_{18:1}$, 0-1% $C_{18:2}$, 4-8% $C_{20:0}$, 33-37% $C_{22:0}$, 0-1% $C_{22:1}$, 0-2% $C_{24:0}$. Free fatty acid content is 0.01-0.1% and Lovibond red color is about 1.0.

The rapeseed/cottonseed oil is converted into methyl esters through an esterification process in which the oil is mixed with methanol, a sodium methoxide catalyst is added, and the reaction is continued until all the triglycerides are converted into methyl esters. Glycerine is settled by gravity after the reaction is completed. The esters are then water washed with hot water to remove trace levels of glycerine and soap. The water phase is settled out by gravity after each wash.

The esters are flash distilled in a batch mode to both remove unsaponifiable materials and to obtain a more concentrated $C_{22}$ material. The distillation is done under a vacuum of 0.5-2mm Hg and a temperature of 300-410°F. The last 10%-15% of the esters distilled are collected into a clean vessel for use in making the desired sucrose polyester. The other 85-90% is discarded. The ester composition of the last 10-15% collected is: 4% $C_{18:0}$, 6% $C_{20:0}$, 87% $C_{22:0}$, 3% $C_{24:0}$. These are esters "A".

Refined and bleached sunflower oil is deodorized at a temperature of 375-495°F under vacuum. The deodorized sunflower oil has the following characteristics: Iodine Value: 125-140; fatty acid composition: 5-10% $C_{16:0}$, 2-6% $C_{18:0}$, 19-26% $C_{18:1}$, 63-74% $C_{18:2}$, 0-2% $C_{18:3}$, 0-1% $C_{20:0}$, 0-1% $C_{22:0}$. Free fatty acid content is 0.01-0.1% and Lovibond red color is about 1.3

The sunflower oil is converted into methyl esters through the same esterification process as described above. The esters are flash distilled in a batch mode, primarily to remove unsaponifiable materials. The distillation is done under a vacuum of 0.5-2.0 mm Hg and a temperature of 300-410°F. These are esters "B".

About 70.5 Kg of methyl esters of refined soybean oil fatty acid, hardened to an IV of about 2, are mixed with 209 Kg of methanol and 15.4 Kg of potassium hydroxide in a stainless steel batch reactor. The mixture is heated to about 145°F (63°C) with agitation for 1 to 3 hours at atmospheric pressure. During this time, all but a residual amount of the methyl esters are saponified to make soap.

About 1193.6 Kg of ester "A" is blended with 241.4 Kg of ester "B" to make ester blend "C". The ester composition of blend "C" is about: 1.2% $C_{16:0}$, 3.8% $C_{18:0}$, 3.8% $C_{18:1}$, 10.7% $C_{18:2}$, 4.7% $C_{20:0}$, 71.9% $C_{22:0}$, 3% $C_{24:0}$. About 545.5 Kg. of ester "C" are added to the previously made soap mixture.

About 104.5 Kg of granular sucrose is then added to give a 5:1 molar ratio of methyl ester to sucrose. Potassium carbonate is then added to the mixture (approx. 0.5 wt. percent of the reaction mix) to catalyze the transesterification . This mixture is agitated and slowly heated at atmospheric pressure until the temperature reaches about 275°F (135°C). This is to remove the methanol. A vacuum is then pulled and the mixture agitated for up to 8 hours to form the mono-, di- and trisucrose esters. Small quantities of tetra- and pentaesters are also formed during this stage. Additional methyl ester "C" (890 Kg) which has been preheated to 275°F (135°C) is added to bring and maintain the molar ratio of the esters to sucrose to 14-15:1. Additional potassium carbonate is then added twice to the mixture (each addition being approximately 0.5 wt. percent of the initial reaction mix). When the reaction conditions stabilize at 275°F (135°C), a nitrogen sparge is used to improve agitation and promote methanol stripping. This second reaction stage lasts approximately 4 to 13 hours.

The reaction mixture is then cooled under nitrogen to between 149°F (65°C) and 185°F (85°C). The crude reaction mixture is agitated with about 91 Kg water. The hydrated crude reaction mixture is passed through a centrifuge to separate a heavy and a light phase. The heavy phase which contains the soaps, excess sugars and potassium carbonate is discarded. The light phase was then washed with an additional 264 Kg of water.

The light phase, which contains methyl esters and the sucrose polyester is then dried to remove moisture at 170°F-190°F (76°-88°C) under 70 mm Hg or less vacuum for 30 to 60 minutes. Filtrol 105 (1.0 wt. percent) is added and the mix is agitated at 167°F (75°C) to 190°F (88°C). The slurry is separated by filtration or other means until there is less than 0.1 wt. percent fines. The liquid is then passed through a 1 micromillimeter filter.

The refined and bleached reaction mix is then passed through a stainless steel wiped-film evaporator or other suitable equipment to distill off the bulk of the methyl esters. The distillation takes place at 392°F (200°C) to 455°F (235°C) under approximately 0.5 mm Hg of vacuum.

The sucrose polyester is then deodorized by passing downward through a stainless steel packed column deodorizer or other suitable device at 392°F (200°C) to 450°F (232°C) under a vacuum of about <25 mm Hg. Steam is introduced to the bottom of the column and passes counter-currently to the sucrose polyester. Feed rates and temperature are adjusted until the methyl ester content of the sucrose polyester is below 1000 ppm. The mixture is then cooled to between 149°F (65°C) to 185°F (85°C) add passed through a 1 micromillimeter filter. The sucrose polyester is stored in clean stainless steel drums.

Sucrose polyester made according to this procedure has the following approximate composition and properties:

| Fatty Acid Composition | |
|---|---|
| $C_{16}$ | 1.2% |
| $C_{17}$ | 0 |
| $C_{16:1}$ | 0 |
| $C_{18}$ | 4.6 |
| $C_{18:1}$ | 3.7 |
| $C_{18:2}$ | 10.9 |
| $C_{18:3}$ | 0 |
| $C_{20}$ | 4.6 |
| $C_{20:1}$ | 0 |
| $C_{22}$ | 71.7 |
| $C_{22:1}$ | 0.2 |
| $C_{24}$ | 2.8 |
| Other | 0.4 |
| Iodine Value | 22.4 |
| Complete Melting Point (By Differential Scanning Calorimetry) | 70.4°C |
| Ester Distribution | |
| Octa | 71.6% |
| Hepta | 28.2 |
| Hexa | 0.2 |
| Penta | <0.1 |
| Lower | <0.1 |

By varying the fatty acid composition of ester "A" and/or ester "B", and/or varying the ratio of ester "A" and ester "B" in preparing ester "C", this process can be used to make other solid sucrose polyol polyesters of the invention.

Complete melting point by DSC is determined as follows:

Equipment:

Perkin-Elmer 7 Series Thermal Analysis System, Model DSC7, manufactured by Perkin-Elmer, Norwalk, CT.

Procedure:

1. Sample is heated to at least 10°C above the complete melt point and mixed thoroughly.
2. 10± 2 mg of sample is weighed into sample pan.
3. A scan is performed from about 10°C above the complete melt point to -60°C at 5°C per minute.
4. The temperature of the sample is maintained at -60°C for 3 minutes and scanned from -60°C to the original starting temperature at 5°C per minute (i.e., about 10°C above the complete melt point).
5. The complete melt point is the temperature at the intersection of the baseline (specific heat line) with the line tangent to the trailing edge of the endothermic peak.

EXAMPLE III

Preparation of a Shortening from the Solid Polyol Polyester of Example I and a Liquid Sucrose Polyester

A noncaloric fat composition suitable for use as a solid shortening is prepared as follows:

Six grams of a solid sucrose polyester prepared according to Example I, and 24 grams of a liquid sucrose polyester, in which the sucrose is substantially completely esterified with fatty acid groups of soybean oil which are hydrogenated to an iodine value of about 107, are mixed and heated until all solids are dissolved. The mixture is allowed to cool back to room temperature to form a plastic composition consisting of 20% solid sucrose polyester of Example I and 80% liquid sucrose polyester. The composition is suitable for use as a food fat, and does not produce the anal leakage problem which would otherwise result if only the liquid sucrose polyester is used as a food fat.

The shortening composition can be treated in the conventional manner with air or nitrogen to form an "aerated" shortening.

EXAMPLE IV

Preparation of Shortening from a Solid Polyol Polyester of Example II, Liquid Sucrose Polyester, and Triglycerides

A high quality, reduced calorie, plastic shortening especially suitable for household use in cooking/frying applications is prepared according to the following formula.

| Ingredient | % |
|---|---|
| Solid sucrose fatty acid polyester (prepared according to the method of Example II) | 1.05 |
| Liquid digestible oil (soybean oil with an Iodine Value of 107) | 43.10 |
| Intermediate melting fraction triglyceride (cotton/soy oil hydrogenated to an iodine value of 48) | 12.60 |
| Hardstock (cotton stearin hardened to an Iodine Value of 1) | 6.30 |
| Liquid sucrose fatty acid polyester nondigestible oil | 33.95 |
| Emulsifier (mono/diglycerides) | 3.00 |
| | 100.00 |

The above ingredients have the following attributes:

| Fatty Acid Content | Solid Sucrose Polyester % | Liquid Sucrose Polyester % |
|---|---|---|
| C12 | - | - |
| C14 | - | - |
| C16 | 1.2 | 9.7 |
| C18 | 4.6 | 5.9 |
| C18:1 | 3.7 | 64.5 |
| C18:2 | 10.9 | 18.9 |
| C18:3 | 0 | 0.2 |
| C17 | 0 | 0.1 |
| C20 | 4.6 | 0.3 |
| C22 | 71.7 | 0.2 |
| C22:1 | 0.2 | 0.2 |
| C24 | 2.8 | - |
| Other | 0.4 | 0.2 |
| Ester Distribution | | |
| Octa | 71.6 | 78.7 |
| Hepta | 28.2 | 21.0 |
| Hexa | 0.2 | 0.2 |
| Lower | <0.2 | 0.3 |

The above ingredients are plasticized by a freeze/pick process, and nitrogen gas is dispersed in the shortening for appearance. The shortening is tempered at 85°F for 24 hours, then stored at 70°F (21°).

EXAMPLE V

Preparation of Salad Oil from a Solid Polyol Polyester, Liquid Sucrose Polyester, and Triglyceride

A substantially clear salad oil is prepared according to the following formula:

| Ingredient | Weight % |
|---|---|
| Solid sucrose fatty acid polyester (prepared according to the acid chloride route) | 0.35 |
| Liquid sucrose fatty acid polyester nondigestible oil | 34.65 |
| Liquid triglyceride digestible oil (unhardened canola) | 65.00 |
| | 100.00 |

The sucrose polyesters have the following attributes:

| Fatty Acid Content | Solid Sucrose Polyester % | Liquid Sucrose Polyester % |
|---|---|---|
| C6 | .55 | - |
| C8 | .24 | - |
| C10 | - | - |
| C13 | .04 | - |
| C14 | .04 | - |
| C16 | .84 | 9.1 |
| C17 | 0.5 | 0.1 |
| C18 | 1.3 | 6.4 |
| C18:1 | 16.63 | 64.4 |
| C18:2 | 0.10 | 18.9 |
| C18:3 | - | 0.3 |
| C20 | 4.05 | 0.3 |
| C22 | 74.17 | 0.2 |
| C24 | 0.5 | - |
| Other | 1.05 | 0.2 |

The ingredients are mixed at approximately 80°C and are then cooled to room temperature. The product is then deaerated to insure no air bubbles are trapped within the product.

EXAMPLE VI

Mayonnaise Composition

A mayonnaise composition of the present invention is made according to the following formula:

| Ingredient | % By Weight |
|---|---|
| Egg Yolk | 8.0 |
| Vinegar | 11.0 |
| Sugar | 2.0 |
| Salt | 1.3 |
| Liquid sucrose octaester of partially hydrogenated soybean oil (I.V. 85) | 66.0 |
| Solid sucrose octaester of mixture of fatty acids from high oleic sunflower oil:behenic acid, 4:4 molar ratio | 11.7 |
|  | 100.0 |

EXAMPLE VII

Margarine Composition

A margarine composition of the present invention is made according to the following formula:

| Ingredient | % By Weight |
|---|---|
| Liquid sucrose octaester of partially hydrogenated (I.V. 85) soybean oil | 68.0 |
| Solid sucrose octaester of mixture of fatty acids from high oleic sunflower oil:behenic acid in 2:6 molar ratio | 12.0 |
| Milk solids | 2.0 |
| Salt | 2.0 |
| Fatty monoglyceride | 1.0 |
| Water | 15.0 |
| | 100.0 |

EXAMPLE VIII

Frying Fat Composition for Potato Chips

A frying fat composition which is particularly suitable for frying potato chips has the following composition:

| Ingredient | Weight % |
|---|---|
| Solid sucrose polyester of the invention | 1.8 |
| Liquid sucrose polyester | 58.2 |
| Cottonseed oil | 40.0 |

The solid ester is prepared by esterification of sucrose with (i) high oleic sunflower oil methyl esters and (ii) methyl esters of a mixture of fatty acids containing about 88.5% behenic. The molar ratio of (i) to (ii) is about 2:6. The solid SPE has the following approximate fatty acid composition: $C_{16:0}$ 0.9%; $C_{18:0}$ 1.3%; $C_{18-1}$ 16.7%, $C_{18:2}$ 1.6%; $C_{20:0}$ 4.6% $C_{22:0}$ 72.3%; $C_{24:0}$ 1.9%. Ester content is approximately: octa 82.6%; hepta 17.1%; hexa 0.1%; lower <0.1%. The liquid sucrose polyester is obtained by esterifying sucrose with methyl esters of soybean oil fatty acids, hardened to an iodine value of about 80. The ester content is about 91.9% octa, 8.1% hepta; <0.1% hexa; <0.1% penta; and <0.1% lower.

The composition is prepared by adding the solid sucrose polyester to a mixture of the heated liquid sucrose polyester and cottonseed oil, mixing until the solid has dissolved, and then cooling.

Approximately 225 Norchip potato slices which have a thickness of about 0.052 inches (.13 cm) are fried in the fat composition in a 5 pound oil capacity batch fryer at a temperature of 365°F (185°C) for 3 minutes, 5 seconds. The fried chips have excellent taste and mouthfeel.

EXAMPLE IX

A cooking oil of the present invention is prepared having the following formula:

| Ingredient | Weight % |
|---|---|
| Solid sucrose fatty acid polyester (prepared according to the method of Example II) | 1.05 |
| Liquid sucrose fatty acid polyester nondigestible oil | 33.95 |
| Liquid triglyceride digestible oil unhardened canola | 65.00 |
| | 100.00 |

The sucrose polyesters have the following attributes:

| Fatty Acid Content | Solid Sucrose Polyester | Liquid Sucrose Polyester |
|---|---|---|
| C10 | - | - |
| C12 | - | - |
| C16 | 1.2 | 10.0 |
| C16-1 | - | 0.1 |
| C18 | 4.6 | 8.0 |
| C18:1 | 3.7 | 69.1 |
| C18:2 | 10.9 | 11.1 |
| C18:3 | 0 | 0.2 |
| C20 | 4.6 | 0.3 |
| C20-1 | - | 0.3 |
| C22 | 71.7 | 0.2 |
| C22:1 | 0.2 | - |
| C24 | 2.8 | - |
| Other | 0.4 | 0.7 |

| Ester Distribution | Solid Sucrose Polyester | Liquid Sucrose Polyester |
|---|---|---|
| Octa | 71.6 | 91.0 |
| Hepta | 28.2 | 9.0 |
| Hexa | 0.2 | <.1 |
| Penta | <0.1 | <0.1 |
| Lower | <0.1 | <0.1 |

The ingredients are mixed at approximately 80°C and then cooled in a scraped wall heat exchanger outlet temperature of about 17°F (-8.3°C). The composition is then deaerated to insure no air bubbles are trapped within the composition.

14

**Claims**

1. A polyol fatty acid polyester having a complete melting point above 25°C wherein:

   a) the polyol has at least 4 hydroxyl groups,
   b) the ester groups consist essentially of (i) $C_{12}$ or higher unsaturated fatty acid radicals or a mixtures of said unsaturated radicals and $C_2$ to $C_{12}$, preferably $C_8$ to $C_{12}$, saturated fatty acid radicals, and (ii) $C_{20}$ or higher saturated fatty acid radicals, the molar ratio of (i):(ii) is from 3:5 to 2:1, preferably 3:5 to 4:4, and
   c) at least 4 of the hydroxyl groups of the polyol are esterified.

2. The polyol fatty acid polyester of Claim 1 wherein: the polyol is a sugar or sugar alcohol having from 4 to 8, preferably 6 to 8, hydroxyl groups, the unsaturated fatty acid radicals of b)(i) have from 12 to 26 carbon atoms, and the saturated fatty acid radicals of b)(ii) have from 20 to 26 carbon atoms.

3. The polyol fatty acid polyester of Claim 2 wherein the polyol is sucrose.

4. The polyol polyester of Claim 3 wherein the fatty acid radicals of b)(i) consist essentially of $C_{18}$ mono- and/or diunsaturated fatty acid radicals.

5. The polyol polyester of Claim 4 wherein the saturated fatty acid radicals of b)(ii) consist essentially of behenic acid radicals.

6. The polyol fatty acid polyester of any one of Claims 1 through 5 wherein the complete melting point of the polyol polyester is above 37°C.

7. A food composition comprising:

   I. a nondigestible edible oil having a complete melting point below 37°C, and
   II. a solid polyol fatty acid polyester having a complete melting point above 37°C, wherein:

   a) the polyol has at least 4 hydroxyl groups,
   b) the ester groups comprise (i) $C_{12}$ or higher unsaturated fatty acid radicals or a mixture of said unsaturated radicals and $C_2$ to $C_{12}$, preferably $C_8$ to $C_{12}$, saturated fatty acid radicals, and (ii) $C_{20}$ or higher saturated fatty acid radicals, the molar ratio of (i):(ii) is from about 1:15 to about 2:1, wherein at least about 15%, preferably at least about 50%, by weight of the fatty acid radicals in b)(ii) are $C_{20}$ or higher saturated fatty acid radicals, and
   c) at least 4 of the hydroxyl groups of the polyol are esterified,

   wherein the weight ratio of I to II is from 99:1 to 1:1, preferably 99:1 to 3:1, more preferably 99:1 to 9:1, and wherein the slope of the SFC profile of the mixture of I and II between 37°C and 21.1°C is between 0.0 and -0.75.

8. The food composition of Claim 7 wherein in II the polyol is a sugar or sugar alcohol having from 4 to 8, preferably 6 to 8, hydroxyl groups, the unsaturated fatty acid radicals of b)(i) have from 12 to 26 carbon atoms, the saturated fatty acid radicals of b)(ii) have from 20 to 26 carbon atoms, the molar ratio of (i) to (ii) is from 1:7 to 5:3, preferably 1:7 to 3:5, and wherein at least 30%, preferably at least about 50%, more preferably at least 60% by weight of the fatty acid radicals in b)(ii) are $C_{20}$ to $C_{26}$ saturated fatty acid radicals.

9. The composition of Claims 8 or 7 wherein the liquid non-digestible oil is a sucrose fatty acid polyester.

10. The composition of Claims 9 or 8 wherein the polyol of the solid polyol polyester is sucrose.

11. The composition of Claims 7 through 11 wherein in b)(i) the fatty acid groups of the solid polyol polyester consist essentially of $C_{18}$ mono- and/or diunsaturated fatty acid radicals.

12. The composition of Claims 7 through 11 wherein in b)(ii) the saturated fatty acid groups of the solid polyol polyester consist essentially of behenic acid radicals.

13. A food composition comprising:

I. a digestible edible oil having a complete melting point below 37°C, and

II. a solid polyol fatty acid polyester having a complete melting point above 25°C, wherein:

a) the polyol has at least 4 hydroxyl groups,

b) the ester groups comprise (i) $C_{12}$ or higher unsaturated fatty acid radicals or a mixture of said unsaturated radicals and $C_2$ to $C_{12}$, preferably $C_8$ to $C_{12}$, saturated fatty acid radicals, and (ii) $C_{20}$ or higher saturated fatty acid radicals, the molar ratio of (i):(ii) is from 1:15 to 2:1, wherein at least 15%, preferably at least 50%, by weight of the fatty acid radicals in b)(ii) are $C_{20}$ or higher saturated fatty acid radicals, and

c) at least 4 of the hydroxyl groups of the polyol are esterified,

wherein the weight ratio of I to II is from 99:1 to 1:1, preferably 99:1 to 3:1, more preferably 99:1 to 9:1.

14. The food composition of Claim 13 wherein in I the digestible oil is a triglyceride and in II the polyol is a sugar or sugar alcohol having from 4 to 8, preferably 6 to 8, hydroxyl groups, the unsaturated fatty acid radicals of b)(i) have from 12 to 26 carbon atoms, the saturated fatty acid radicals of b)(ii) have from 20 to 26 carbon atoms, the molar ratio of (i) to (ii) is from 1:7 to 5:3; and wherein at least 30%, preferably at least 60% by weight of the fatty acid radicals in b)(ii) are $C_{20}$ to $C_{26}$ saturated fatty acid radicals.

15. The composition of Claims 13 or 14 wherein the polyol of the solid polyol polyester is sucrose.

16. The composition of Claims 13 through 15 wherein in b)(i) the fatty acid groups of the solid polyol polyester consist essentially of $C_{18}$ mono- and/or diunsaturated fatty acid radicals.

17. The composition of Claims 13 through 16 wherein in b)(ii) the saturated fatty acid groups of the solid polyol polyester consist essentially of behenic acid radicals.

## Patentansprüche

1. Polyolfettsäurepolyester mit einem über 25°C liegenden Schmelzpunkt für das vollständige Schmelzen, worin:

a) das Polyol wenigstens 4 Hydroxylgruppen aufweist;

b) die Estergruppen im wesentlichen aus (i) $C_{12}$- oder höheren ungesättigten Fettsäureresten oder einem Gemisch dieser ungesättigten Reste und $C_2$- bis $C_{12}$-, vorzugsweise $C_8$- bis $C_{12}$- gesättigten Fettsäureresten und (ii) $C_{20}$- oder höheren gesättigten Fettsäureresten bestehen, wobei das Molverhältnis von (i) zu (ii) von 3:5 bis 2:1, vorzugsweise von 3:5 bis 4:4 beträgt, und

(c) wenigstens 4 der Hydroxylgruppen des Polyols verestert sind.

2. Polyolfettsäurepolyester nach Anspruch 1, worin das Polyol ein Zucker oder Zuckeralkohol mit 4 bis 8, vorzugsweise 6 bis 8 Hydroxylgruppen ist, die ungesättigten Fettsäurereste von b) (i) 12 bis 26 Kohlenstoffatome aufweisen und die gesättigten Fettsäurereste von b) (ii) 20 bis 26 Kohlenstoffatome aufweisen.

3. Polyolfettsäurepolyester nach Anspruch 2, worin das Polyol Saccharose ist.

4. Polyolpolyester nach Anspruch 3, worin die Fettsäurereste von b) (i) im wesentlichen aus $C_{18}$- einfach - und/oder zweifach ungesättigten Fettsäureresten bestehen.

5. Polyolpolyester nach Anspruch 4, worin die gesättigten Fettsäurereste von b) (ii) im wesentlichen aus Behensäureresten bestehen.

6. Polyolfettsäurepolyester nach einem der Ansprüche 1 bis 5, worin der Schmelzpunkt für das vollständige Schmelzen des Polyolpolyesters über 37°C liegt.

7. Nahrungsmittelzusammensetzung, umfassend:

I. ein unverdauliches eßbares Öl mit einem unter 37°C liegenden Schmelzpunkt für das vollständige Schmelzen

und

II. einen festen Polyolfettsäureester mit einem über 37°C liegenden Schmelzpunkt für das vollständige Schmelzen, worin:

a) das Polyol wenigstens 4 Hydroxylgruppen aufweist,
b) die Estergruppen (i) $C_{12}$- oder höhere ungesättigte Fettsäurereste oder ein Gemisch aus diesen ungesättigten Resten und $C_2$- bis $C_{12}$-, vorzugsweise $C_8$- bis $C_{12}$- gesättigten Fettsäureresten und (ii) $C_{20}$- oder höhere gesättigte Fettsäurereste umfassen, wobei das Molverhältnis von (i) zu (ii) von etwa 1:15 bis etwa 2:1 beträgt, wobei wenigstens etwa 15 %, vorzugsweise wenigstens etwa 50 Gew.-% der Fettsäurereste in b) (ii) $C_{20}$- oder höhere gesättigte Fettsäurereste sind, und
c) wenigstens 4 der Hydroxylgruppen des Polyols verestert sind,

worin das Gewichtsverhältnis von I zu II von 99:1 bis 1:1, vorzugsweise 99:1 bis 3:1, stärker bevorzugt 99:1 bis 9:1 beträgt, und worin der Anstieg des SFC-Profils des Gemisches aus I und II zwischen 37°C und 21,1°C zwischen 0,0 und -0,75 beträgt.

8. Nahrungsmittelzusammensetzung nach Anspruch 7, worin in II das Polyol ein Zucker oder ein Zuckeralkohol mit 4 bis 8, vorzugsweise 6 bis 8 Hydroxylgruppen ist, die ungesättigten Fettsäurereste von b) (i) von 12 bis 26 Kohlenstoffatome aufweisen, die gesättigten Fettsäurereste von b) (ii) von 20 bis 26 Kohlenstoffatome aufweisen, das Molverhältnis von (i) zu (ii) von 1:7 bis 5:3, vorzugsweise 1:7 bis 3:5 beträgt, und worin wenigstens 30 %, vorzugsweise wenigstens etwa 50 %, stärker bevorzugt wenigstens 60 Gew.-% der Fettsäurereste in b) (ii) $C_{20}$- bis $C_{26}$- gesättigte Fettsäurereste sind.

9. Zusammensetzung nach Anspruch 8 oder 7, worin das flüssige unverdauliche Öl ein Saccharosefettsäurepolyester ist.

10. Zusammensetzung nach Anspruch 9 oder 8, worin das Polyol des festen Polyolpolyesters Saccharose ist.

11. Zusammensetzung nach den Ansprüchen 7 bis 10, worin in b) (i) die Fettsäuregruppen des festen Polyolpolyesters im wesentlichen aus $C_{18}$- einfach und/oder zweifach ungesättigten Fettsäureresten bestehen.

12. Zusammensetzung nach den Ansprüchen 7 bis 11, worin in b) (ii) die gesättigten Fettsäuregruppen des festen Polyolpolyesters im wesentlichen aus Behensäureresten bestehen.

13. Nahrungsmittelzusammensetzung, umfassend:

I. ein verdauliches eßbares Öl mit einem unter 37°C liegenden Schmelzpunkt für das vollständige Schmelzen, und
II. einen festen Polyolfettsäurepolyester mit einem über 25°C liegenden Schmelzpunkt für das vollständige Schmelzen, worin:

a) das Polyol wenigstens 4 Hydroxylgruppen aufweist,
b) die Estergruppen (i) $C_{12}$- oder höhere ungesättigte Fettsäurereste oder ein Gemisch aus diesen ungesättigten Resten und $C_2$- bis $C_{12}$-, vorzugsweise $C_8$- bis $C_{12}$- gesättigten Fettsäureresten und (ii) $C_{20}$- oder höhere gesättigte Fettsäurereste umfassen, wobei das Molverhältnis von (i) zu (ii) von 1:15 bis 2:1 beträgt, wobei wenigstens 15 %, vorzugsweise wenigstens 50 Gew.-% der Fettsäurereste in b) (ii) $C_{20}$- oder höhere gesättigte Fettsäurereste sind, und
c) Wenigstens 4 der Hydroxylgruppen des Polyols verestert sind,

worin das Gewichtsverhältnis von I zu II von 99:1 bis 1:1, vorzugsweise 99:1 bis 3:1, stärker bevorzugt 99:1 bis 9:1 beträgt.

14. Nahrungsmittelzusammensetzung nach Anspruch 13, worin in I das verdauliche Öl ein Triglycerid ist und in II das Polyol ein Zucker oder ein Zuckeralkohol mit 4 bis 8, vorzugsweise 6 bis 8 Hydroxylgruppen ist, die ungesättigten Fettsäurereste von b) (i) von 12 bis 26 Kohlenstoffatome aufweisen, die gesättigten Fettsäurereste von b) (ii) von 20 bis 26 Kohlenstoffatome aufweisen, das Molverhältnis von (i) zu (ii) von 1:7 bis 5:3 beträgt und worin wenigstens 30 %, vorzugsweise wenigstens 60 Gew.-% der Fettsäurereste in b) (ii) $C_{20}$- bis $C_{26}$- gesättigte Fettsäurereste sind.

15. Zusammensetzung nach Anspruch 13 oder 14, worin das Polyol des festen Polyolpolyesters Saccharose ist.

**16.** Zusammensetzung nach den Ansprüchen 13 bis 15, worin in b) (i) die Fettsäuregruppen des festen Polyolpolyesters im wesentlichen aus $C_{18}$- einfach und/oder zweifach ungesättigten Fettsäureresten bestehen.

**17.** Zusammensetzung nach den Ansprüchen 13 bis 16, worin in b) (ii) die gesättigten Fettsäuregruppen des festen Polyolpolyesters im wesentlichen aus Behensäureresten bestehen.

## Revendications

**1.** Polyester d'acide gras et de polyol ayant un point de fusion finale supérieur à 25°C, dans lequel :

a) le polyol comporte au moins 4 groupes hydroxyle,
b) les groupes ester sont constitués essentiellement: (i) de radicaux acide gras insaturés en $C_{12}$ ou plus, ou d'un mélange desdits radicaux insaturés et de radicaux acide gras saturés en $C_2$ à $C_{12}$, de préférence en $C_8$ à $C_{12}$, et (ii) de radicaux acide gras saturés en $C_{20}$ ou plus, le rapport molaire (i)/(ii) étant compris entre 3/5 et 2/1, de préférence entre 3/5 et 4/4, et
c) au moins 4 des groupes hydroxyle du polyol sont estérifiés.

**2.** Polyester d'acide gras et de polyol selon la revendication 1, dans lequel le polyol est un sucre ou un alcool de sucre ayant de 4 à 8 et de préférence de 6 à 8 groupes hydroxyle, les radicaux acide gras insaturés du paragraphe (b)(i) ont de 12 à 26 atomes de carbone, et les radicaux acide gras saturés du paragraphe (b)(ii) ont de 20 à 26 atomes de carbone.

**3.** Polyester d'acide gras et de polyol selon la revendication 2, dans lequel le polyol est le saccharose.

**4.** Polyester de polyol selon la revendication 3, dans lequel les radicaux acide gras du paragraphe (b)(i) sont constitués essentiellement de radicaux acide gras di-insaturés et/ou mono-insaturés en $C_{18}$.

**5.** Polyester de polyol selon la revendication 4, dans lequel les radicaux acide gras saturés du paragraphe (b)(ii) sont constitués essentiellement de radicaux acide béhénique.

**6.** Polyester d'acide gras et de polyol selon l'une quelconque des revendications 1 à 5, dans lequel le point de fusion finale du polyester de polyol est supérieur à 37°C.

**7.** Composition alimentaire comprenant :

I) une huile comestible non digestible ayant un point de fusion finale inférieur à 37°C, et
II) un polyester d'acide gras et de polyol solide ayant un point de fusion finale supérieur à 37°C, dans lequel :

a) le polyol comporte au moins 4 groupes hydroxyle,
b) les groupes ester sont constitués: (i) de radicaux acide gras insaturés en $C_{12}$ ou plus, ou d'un mélange desdits radicaux insaturés et de radicaux acide gras saturés en $C_2$ à $C_{12}$, de préférence en $C_8$ à $C_{12}$, et (ii) de radicaux acide gras saturés en $C_{20}$ ou plus, le rapport molaire (i)/(ii) étant compris entre environ 1/15 et environ 2/1,

dans lequel au moins environ 15 % et de préférence au moins environ 50 % en poids des radicaux acide gras du paragraphe (b)(ii) sont des radicaux acide gras saturés en $C_{20}$ ou plus, et

c) au moins 4 des groupes hydroxyle du polyol sont estérifiés,

dans laquelle le rapport pondéral de I) à II) est compris entre 99/1 et 1/1, de préférence entre 99/1 et 3/1, mieux encore entre 99/1 et 9/1, et dans laquelle la pente de la courbe SFC du mélange de I) et II) entre 37°C et 21,1°C est comprise entre 0,0 et -0,75.

**8.** Composition alimentaire selon la revendication 7, dans laquelle, dans le paragraphe II), le polyol est un sucre ou un alcool de sucre ayant de 4 à 8 et de préférence de 6 à 8 groupes hydroxyle, les radicaux acide gras insaturés du paragraphe (b)(i) ont de 12 à 26 atomes de carbone, les radicaux acide gras saturés du paragraphe (b)(ii) ont de 20 à 26 atomes de carbone, le rapport molaire de (i) à (ii) est compris entre 1/7 et 5/3, de préférence entre 1/7 et 3/5, et dans laquelle au moins 30 %, de préférence au moins environ 50 % et mieux encore au moins 60 % en poids des radicaux acide gras du paragraphe b)(ii) sont des radicaux acide gras saturés en $C_{20}$ à $C_{26}$.

9. Composition selon la revendication 8 ou 7, dans laquelle l'huile non digestible liquide est un polyester d'acide gras et de saccharose.

10. Composition selon la revendication 9 ou 8, dans laquelle le polyol du polyester de polyol solide est le saccharose.

11. Composition selon les revendications 7 à 11, dans laquelle, dans le paragraphe (b)(i), les groupes acide gras du polyester de polyol solide sont constitués essentiellement de radicaux acide gras di-insaturés et/ou mono-insaturés en $C_{18}$.

12. Composition selon les revendications 7 à 11, dans laquelle, dans le paragraphe (b)(ii), les groupes acide gras saturés du polyester de polyol solide sont constitués essentiellement de radicaux acide béhénique.

13. Composition alimentaire comprenant :

   I) une huile comestible digestible ayant un point de fusion finale inférieur à 37°C, et
   II) un polyester d'acide gras et de polyol solide ayant un point de fusion finale supérieur à 25°C, dans lequel :

   a) le polyol comporte au moins 4 groupes hydroxyle,
   b) les groupes ester sont constitués: (i) de radicaux acide gras insaturés en $C_{12}$ ou plus, ou d'un mélange desdits radicaux insaturés et de radicaux acide gras saturés en $C_2$ à $C_{12}$, de préférence en $C_8$ à $C_{12}$, et (ii) de radicaux acide gras saturés en $C_{20}$ ou plus, le rapport molaire (i)/(ii) étant compris entre 1/15 et 2/1,

   dans lequel au moins 15 % et de préférence au moins 50 % en poids des radicaux acide gras du paragraphe (b)(ii) sont des radicaux acide gras saturés en $C_{20}$ ou plus, et

   c) au moins 4 des groupes hydroxyle du polyol sont estérifiés,

   dans laquelle le rapport pondéral de I) à II) est compris entre 99/1 et 1/1, de préférence entre 99/1 et 3/1, mieux encore entre 99/1 et 9/1.

14. Composition alimentaire selon la revendication 13, dans laquelle, dans le paragraphe I), l'huile digestible est un triglycéride et, dans le paragraphe II), le polyol est un sucre ou un alcool de sucre ayant de 4 à 8 et de préférence de 6 à 8 groupes hydroxyle, les radicaux acide gras insaturés du paragraphe (b)(i) ont de 12 à 26 atomes de carbone, les radicaux acide gras saturés du paragraphe (b)(ii) ont de 20 à 26 atomes de carbone, le rapport molaire de (i) à (ii) est compris entre 1/7 et 5/3, et dans laquelle au moins 30 % et de préférence au moins 60 % en poids des radicaux acide gras du paragraphe b)(ii) sont des radicaux acide gras saturés en $C_{20}$ à $C_{26}$.

15. Composition selon la revendication 13 ou 14, dans laquelle le polyol du polyester de polyol solide est le saccharose.

16. Composition selon les revendications 13 à 15, dans laquelle, dans le paragraphe (b)(i), les groupes acide gras du polyester de polyol solide sont constitués essentiellement de radicaux acide gras di-insaturés et/ou mono-insaturés en $C_{18}$.

17. Composition selon les revendications 13 à 16, dans laquelle, dans le paragraphe (b)(ii), les groupes acide gras saturés du polyester de polyol solide sont constitués essentiellement de radicaux acide béhénique.